Europäisches Patentamt

European Patent Office

Office européen des brevets

(11) Veröffentlichungsnummer : **0 021 337**
**B1**

(12) **EUROPÄISCHE PATENTSCHRIFT**

(45) Veröffentlichungstag der Patentschrift :
07.12.83

(21) Anmeldenummer : 80103395.2

(22) Anmeldetag : 18.06.80

(51) Int. Cl.³ : **A 61 K   9/50**, A 61 K 31/55

(54) Präparate zur parenteralen Verabreichung fettlöslicher Wirkstoffe.

(30) Priorität : 22.06.79 CH 5863/79

(43) Veröffentlichungstag der Anmeldung :
07.01.81 Patentblatt 81/01

(45) Bekanntmachung des Hinweises auf die Patenterteilung : 07.12.83 Patentblatt 83/49

(84) Benannte Vertragsstaaten :
AT BE CH DE FR GB IT LI LU NL SE

(56) Entgegenhaltungen :
DE-A- 2 818 655
FR-A- 2 081 586
FR-A- 2 298 318
GB-A- 2 002 319
CHEMICAL ABSTRACTS, Band 90, Nr. 4, 22-12-1979,
Seite 169 Zusammenfassung 35233p Columbus,
Ohio, USA SCHACHTER, D:"Aggregation of liposomes by Dextrans of high molecular weight"
CHEMICAL ABSTRACTS, Band 78, Nr. 20, 21-05-1973,
Seite 265, Zusammenfassung 128390e Columbus,
Ohio, USA CHOWHAN, Z et al.: "Model transport
studies utilizing lecithin spherules. III. Transport of
taurocholic acid, tritiated in the cholic acid portion in
buffered D-glucose solutions"

(73) Patentinhaber : **F. HOFFMANN-LA ROCHE & CO.
Aktiengesellschaft
CH-4002 Basel (CH)**

(72) Erfinder : **Schrank, Jürg, Dr.
Im Hirshalm 49
CH-4125 Riehen (CH)**
Erfinder : **Steffen, Hans, Dr.
Olsbergerstrasse 27
CH-4411 Arlsdorf (CH)**

(74) Vertreter : **Lederer, Franz, Dr. et al
Patentanwälte Dr. Franz Lederer Reiner F. Meyer
Lucile-Grahn-Strasse 22
D-8000 München 80 (DE)**

## Präparate zur parenteralen Verabreichung fettlöslicher Wirkstoffe

Eine Anzahl fettlöslicher pharmazeutischer Wirkstoffe liess sich bisher nicht oder nur unter Inkaufnahme unerwünschter Nebenwirkungen parenteral applizieren, weil z. B. Hämolyse, Venenthrombosen oder Koagulation des Bluts auftraten.

Ueberraschenderweise hat sich nun gezeigt, dass gewisse derartige Wirkstoffe parenteral verabreicht werden können, wenn dies in Form einer Liposomen-Lösung geschieht. In dieser Verabreichungsform zeigen die Präparate keine unerwünschten Nebenwirkungen, währen die biologische Aktivität der Wirkstoffe voll erhalten bleibt.

Während die Herstellung pharmazeutisch verwendbarer Liposomen-Lösungen an sich seit langem bekannt ist (siehe z. B. G. Gregoriadis, « Liposomes as Drug Carriers in Biology and Medicine », und Brenda E. Ryman, « Liposome Delivery of Materials of Therapeutic Interest — Possibilities and Problems », in den Abstracts des Symposiums « The Potential of Liposomes as Drug Carriers », 2.-3. März 1978, Battelle-Institut, Genf, Schweiz, S. 5-11 bzw. 26-31), ist die Herstellung langfristig stabiler Liposomen-Lösungen ein Problem, das bis heute noch nicht in zufriedenstellender Weise gelöst werden konnte. Die Stabilität derartiger Lösungen hängt einerseits vom Lipid, andererseits von der Zusammensetzung der wässrigen Phase ab. Auch können bereits geringfügige Verunreinigungen dazu führen, dass es zur vorzeitigen Ausflockung kommt, was für ein pharmazeutisches Präparat nicht in Kauf genommen werden kann. Es hat sich nun gezeigt, dass diese Schwierigkeiten überwunden werden können und sich langfristig stabile Liposomen-Lösungen zur parenteralen Applikation erhalten lassen durch Zusatz von Zuckern, und zwar in relativ grossen Mengen, so dass sie als Schutzkolloide wirken (siehe auch GB-A-2 002 219).

Die vorliegende Erfindung betrifft pharmazeutische Präparate zur parenteralen Applikation von Benzodiazepinen, Dibenzothiepinen oder Chinolinmethanolderivaten, dadurch gekennzeichnet, dass sie den Wirkstoff in Form einer wässrigen Liposomen-Lösung enthalten, deren wässrige Phase einen Gehalt an einem Zucker oder zuckerähnlichen Polyol in mindestens 0,4 molarer Konzentration aufweist ; und daraus hergestellte Lyophilisate. Die Erfindung betrifft ferner die Herstellung solcher Präparate.

Als Liposomenbildner kommen die allgemein bekannten Lipide, insbesonders Phospholipide in Frage, wobei Lecithine bevorzugt sind. Die Lecithine können pflanzlicher, tierischer oder synthetischer Provenienz sein, wie Sojalecithin, Eilecithin oder L-β-Oleoyl-γ-palmitoyl-α-lecithin. Die Liposomen können mono- oder multilamellar aufgebaut sein (für i. v. Applikation sind monolamellare bevorzugt) und besitzen vorzugsweise einen Durchmesser von 25-200 nm.

Benzodiazepine und Chinolinmethanolderivate, die bisher nicht bzw. nicht ohne unerwünschte Nebenwirkungen parenteral applizierbar waren, sind z. B. 7-Chlor-1,3-dihydro-1-methyl-5-phenyl-2H-1,4-benzodiazepin-2-on (Diazepam) 5-(o-Fluorphenyl)-1,3-dihydro-1-methyl-7-nitro-2H-1,4-benzodiazepin-2-on (Flunitrazepam) und erythro-α-2-Piperidyl-2,8-bis-(trifluormethyl)-4-chinolinmethanol (Mefloquin) oder eines seiner Additionssalze mit einer physiologisch verträglichen Säure, wie einer für derartige Zwecke üblichen organischen und anorganischen Säure, z. B. Salzsäure, Schwefelsäure, Phosphorsäure, Ameisensäure, Essigsäure, Oxalsäure, Weinsäure, Maleinsäure, Benzoesäure, Bernsteinsäure, Fumarsäure, Lävulinsäure, Salicylsäure, Citronensäure, Isocitronensäure, Adipinsäure, Milchsäure, α-Ketoglutarsäure, Aepfelsäure, Malonsäure, Glycerinsäure, Mevalonsäure, Glucuronsäure, Neuraminsäure, Glutarsäure, Asparaginsäure, Gluconsäure, Mandelsäure, Ascorbinsäure, Lactobionsäure, Glucoheptonsäure, Glutaminsäure, Nicotinsäure, Panthotensäure, Folsäure, Adenylsäure, Geranylsäure, Cytidinsäure und Inosinsäure. Beispiele von Dibenzothiepinen, die in den erfindungsgemässen Präparaten enthalten sein können sind antidepressiv und neuroleptisch wirksame Dibenzothiepine der deutschen Offenlegungsschrift 2 412 522.

Die Konzentration des Liposomenbildners in der Lösung liegt im Rahmen des allgemein üblichen, d. h. etwa zwischen 1 und 25 % (Gew./Vol.) vorzugsweise zwischen 5 und 15 % (Gew./Vol.), während die des Wirkstoffes weitgehend durch seine biologische Aktivität bestimmt wird. Die konzentrationen dieser beiden Lösungskomponenten sind jedenfalls kein Kriterium für die Grenzen der vorliegenden Erfindung. So kommen beispielsweise für Diazepam 0,1-10 mg/ml, vorzugsweise 5 mg/ml, für Mefloquin beispielsweise 1-30 mg/ml, vorzugsweise 5-20 mg/ml in Betracht.

Als weitere, fakultative Komponenten der erfindungsgemässen Präparate kommen die in derartigen Präparaten üblichen Zusatz- und Hilfsstoffe in Frage d. s. geringere Mengen weiterer Lipide, wie Cholesterin, oder Antioxydantien und Synergisten, Konservierungsmittel, Stabilisierungsmittel, Puffersubstanzen oder Mittel zur Einstellung des gewünschten pH-Wertes oder osmotischen Druckes. Die erforderlichen und optimalen Mengen dieser Zusatz- und Hilfsstoffe können von Fall zu Fall variieren.

Essentieller Bestandteil der erfindungsgemässen Präparate ist jedoch ein Zucker, durch den die notwendige Stabilität erreicht wird. Als Zuckerkomponente kommen die üblichen Mono- und Disaccharide bzw. zuckerähnlichen Polyole in Frage, wie Glucose, Fructose, Saccharose, Sorbit oder Xylit. Damit der gewünschte Stabilitätseffekt gewährleistet ist, sollte die Konzentration der Zuckerkomponente in der Liposomen-Lösung mindestens 0,4 molar sein, während die obere Grenze nicht kritisch ist und durch die Viskositätsverhältnisse bestimmt wird.

Die erfindungsgemässen Präparate können in an sich bekannter Weise hergestellt werden, z. B. dadurch, dass man den Wirkstoff in wässriger Lösung in Gegenwart eines Liposomenbildners und eines Zuckers sowie ggf. in Gegenwart weiterer, üblicher Zusatzstoffe homogenisiert, oder aus den Wirkstoff und einen Zucker enthaltenden Lecithin-Detergentien-Mischmicellen das Detergens, z. B. mittels Dialyse oder Gelchromatographie, abtrennt. Als Detergentien kommen hierbei insbesondere gallensäuren in Betracht. Die Gelchromatographie kann z. B. an Sephadex G 50 durchgeführt werden ; für die Dialyse eignet sich z. B. Union-Carbide-Dialyseschlauch. Die so erhaltene Liposomenlösung kann sterilisiert und gewünschtenfalls lyophilisiert werden. Die Homogenisierung erfolgt vorzugsweise unter Druck, z. B. 300 Bar und darüber, oder auch mittels Ultraschall. Aus den Lyophilisaten können kurz vor Gebrauch in üblicher Weise durch Zusatz von Wasser oder isotonischer Salzlösung die für die Injektion oder Infusion geeigneten Lösungen rekonstituiert werden.

Die folgenden Beispiele erläutern die Erfindung ohne sie zu begrenzen.

Beispiel 1

1 000 mg Mefloquin-hydrochlorid und 5 g gereinigtes Sojalecithin wurden mit einer Lösung von 0,7 M Saccharose in 0,067 M Phosphatpuffer pH 7 (Na$_2$HPO$_4$/NaH$_2$PO$_4$) zu insgesamt 50 ml gelöst. Die Lösung wurde so lange mit einem Polytronhomogenisator behandelt, bis sie gleichmässig milchig war und keine einzelnen Teilchen mehr mit dem blossen Auge sichtbar waren. Anschliessend wurde etwa 60 Minuten bei einer Temperatur unterhalb von 25 °C und unter Stickstoff mit einem Branson Ultraschallfinger bei etwa 70 W beschallt. Die erhaltenen Liposomen wurden 15 Minuten in einer Zentrifuge sedimentiert (6 000 × g). Nach Sterilfiltration bei 120 °C, 20 Minuten, wurde die Lösung sterilisiert und anschliessend lyophilisiert. Aus dem Lyophilisat wurde durch Zusatz von Wasser eine Injektionslösung rekonstituiert, in der der Teilchendurchmesser der gleiche wie in der Lösung von der Lyophilisation war.

Beispiel 2

25 g gereinigtes Sojalecithin, 62,5 g Saccharose, 825 mg Diazepam und 185 ml mit Stickstoff behandelter 1/15 M Phosphatpuffer pH 7 wurden solange mit einem Polytronhomogenisator behandelt, bis das Gemisch gleichmässig milchig war. Es wurde dann unter einem Druck von 500 Atmosphären bei 20 °C im Umlauf homogenisiert, wobei das Gemisch den Homogenisator insgesamt etwa 60 × durchfloss. Es wurde eine opaleszierende Diazepam-Liposomen-Lösung erhalten, die sterilfiltriert und sterilisiert wurde.

Beispiel 3

5 g gereinigtes Sojalecithin und 50 mg Flunitrazepam wurden in 20 ml Aethanol gelöst. Das Aethanol wurde am Rotationsverdampfer entfernt. Danach wurden 6,37 g Sorbit und 50 mg Natriumpyrosulfit (als Antioxydans) zugesetzt und mit 1/15 M Phosphatpuffer auf 50 ml aufgefüllt.

Beispiel 4

5 g gereinigtes Sojalecithin und 50 mg Flunitrazepam wurden in 20 ml Aethanol gelöst. Das Aethanol wurde am Rotationsverdampfer entfernt. Danach wurden 16 g Glucose und 50 mg Natriumpyrosulfit (als Antioxydans) zugesetzt und mit 1/15 M Phosphatpuffer auf 50 ml aufgefüllt.

**Ansprüche** (für die Vertragsstaaten : BE, CH, DE, FR, GB, IT, LI, LU, NL, SE)

1. Pharmazeutische Präparate zur parenteralen Applikation von Benzodiazepinen, Dibenzothiepinen oder Chinolinmethanolderivaten, dadurch gekennzeichnet, dass sie den Wirkstoff in Form einer wässrigen Liposomen-Lösung enthalten, deren wässrige Phase einen Gehalt an einem Zucker oder zuckerähnlichen Polyol in mindestens 0,4 molarer Konzentration aufweist ; und daraus hergestellte Lyophilisate.

2. Präparate gemäss Anspruch 1, dadurch gekennzeichnet, dass das Benzodiazepin 7-Chlor-1,3-dihydro-1-methyl-5-phenyl-2H-1,4-benzodiazepin-2-on, und das Chinolinmethanolderivat erythro-α-2-Piperidyl-2,8-bis-(trifluormethyl)-4-chinolinmethanol, bzw. eines seiner Additionssalze mit einer physiologisch verträglichen Säure ist.

3. Präparate gemäss einem der Ansprüche 1 oder 2, dadurch gekennzeichnet, dass es sich um monolamellare Liposomen mit einem Durchmesser von 25-200 nm handelt.

**Ansprüche** (für den Vertragsstaat AT)

1. Verfahren zur Herstellung pharmazeutischer Präparate zur parenteralen Applikation von benzodiazepinen, Dibenzothiepinen oder Chinolinmethanolderivaten, dadurch gekennzeichnet, dass man den Wirkstoff in wässriger Lösung in Gegenwart eines Liposomenbildners und eines Zuckers oder zuckerähnlichen Polyols, dessen Konzentration in der wässrigen Phase mindestens 0,4 molar ist, sowie ggf. in Gegenwart weiterer, üblicher Zusatzstoffe homogenisiert und die so erhaltene Liposomenlösung gewünschtenfalls lyophilisiert.

2. Verfahren gemäss Anspruch 1, dadurch gekennzeichnet, dass das Benzodiazepin 7-Chlor-1,3-dihydro-1-methyl-5-phenyl-2H-1,4-benzodiazepin-2-on, und das Chinolinmethanolderivat erythro-α-2-Piperidyl-2,8-bis-(trifluormethyl)-4-chinolinmethanol, bzw. eines seiner Additionssalze mit einer physiologisch verträglichen Säure ist.

3. Verfahren gemäss einem der Ansprüche 1-3, dadurch gekennzeichnet, dass es sich um monolamellar Liposomen mit einem Durchmesser on 25-200 nm handelt.

**Claims** (for the Contracting States : BE, CH, DE, FR, GB, IT, LI, LU, NL, SE)

1. Pharmaceutical preparations for the parenteral administration of benzodiazepines, dibenzothiepines or quinolinemethanol derivatives, characterized in that they contain the active substance in the form of an aqueous liposome solution, the aqueous phase of which contains a sugar or sugar-like polyol in at least 0.4 molar concentration ; and lyophilizates manufactured therefrom.

2. Preparations according to claim 1, characterized in that the benzodiazepine is 7-chloro-1,3-dihydro-1-methyl-5-phenyl-2H-1,4-benzodiazepin-2-one, and the quinolinemethanol derivative is erythro-α-2-piperidyl-2,8-bis-(trifluoromethyl)-4-quinolinemethanol, or one of its acid addition salts with a physiologically compatible acid.

3. Preparations according to one of claims 1 or 2, characterized in that the liposome is a monolamellar liposome having a diameter of 25-200 nm.

**Claims** (for the Contracting State AT)

1. Process for the manufacture of pharmaceutical preparations for the parenteral administration of benzodiazepines, dibenzothiepines or quinolinemethanol derivatives, characterized by homogenizing the active substance in aqueous solution in the presence of a liposome-former and a sugar or sugar-like polyol, the concentration of which in the aqueous phase is at least 0.4 molar, and, if desired, in the presence of other usual additives and, if desired, lyophilizing the thus-obtained liposome solution.

2. Process according to claim 1, characterized in that the benzodiazepine is 7-chloro-1,3-dihydro-1-methyl-5-phenyl-2H-1,4-benzodiazepin-2-one, and the quinolinemethanol derivative is erythro-α-2-piperidyl-2,8-bis-(trifluoromethyl)-4-quinolinemethanol, or one of its acid addition salts with a physiologically compatible acid.

3. Process according to any one of claims 1-3, characterized in that the liposome is a monolamellar liposome having a diameter of 25-200 nm.

**Revendications** (pour les Etats Contractants : BE, CH, DE, FR, GB, IT, LI, LU, NL, SE)

1. Compositions pharmaceutiques pour l'administration parentérale de benzodiazépines, de dibenzothiépines ou de dérivés du quinoléine-méthanol, caractérisées en ce qu'elles contiennent la substance active sous la forme d'une solution aqueuse liposomique dont la phase aqueuse contient un sucre ou un polyol analogue à un sucre à une concentration au moins 0,4 molaire ; et les lyophilisats préparés à partir de telles compositions.

2. Compositions selon la revendication 1, caractérisées en ce que la benzodiazépine est la 7-chloro-1,3-dihydro-1-méthyl-5-phényl-2H-1,4-benzodiazépine-2-one et le dérivé de quinoléine-méthanol est l'érythro-α-2-pipéridyl-2,8-bis-(trifluorométhyl)-4-quinoléine-méthanol ou l'un de ses sels formés par addition avec un acide toléré par l'organisme.

3. Compositions selon l'une des revendications 1 ou 2, caractérisées en ce qu'il s'agit de liposomes mono-lamellaires à un diamètre de 25 à 200 nm.

**Revendications** (pour l'Etat Contractant AT)

1. Procédé de préparation de compositions pharmaceutiques pour l'administration parentérale de benzodiazépines, de dibenzothiépines ou de dérivés du quinoléine-méthanol, caractérisé en ce que l'on homogénéise la substance active en solution aqueuse en présence d'un générateur de liposomes et d'un sucre ou d'un polyol analogue à un sucre dont la concentration dans la phase aqueuse est au moins 0,4 molaire, et éventuellement en présence d'autres additifs usuels, et si on le désire, on lyophilise la solution liposomique ainsi obtenue.

2. Procédé selon la revendication 1, caractérisé en ce que la benzodiazépine est la 7-chloro-1,3-dihydro-1-méthyl-5-phényl-2H-1,4-benzodiazépine-2-one et le dérivé de quinoléine-méthanol l'érythro-α-2-pipéridyl-2,8-bis-(trifluorométhyl)-4-quinoléine-méthanol ou l'un de ses sels formés par addition avec un acide toléré par l'organisme.

3. Procédé selon l'une des revendications 1-2, caractérisé en ce qu'il s'agit de liposomes mono-lamellaires à un diamètre de 25 à 200 nm.